# FASCICULE DE BREVET EUROPEEN

(11) **EP 0 546 875 B1**
(45) Date de publication et mention de la délivrance du brevet: **11.09.1996**
(21) Numéro de dépôt: 92402996.0
(22) Date de dépôt: 05.11.1992
(51) Int. Cl.: C07J 5/00, C07J 41/00

(54) **Nouveau procédé de préparation de dérivés 20-oxo 17alpha,21-dihydroxylés du pregnane et nouveaux intermédiaires**
Neues Verfahren zur Herstellung von 20-Oxo, 17-Alpha, 21-Dihydroxy Derivaten von Pregnan und neue Zwischenprodukte dafür
New process for the preparation of 20-oxo, 17-alpha, 21-dihydroxy derivatives of pregnane and new intermediates thereof

(30) Priorité: 08.11.1991 FR 9113777
(43) Date de publication de la demande: 16.06.1993
(73) Titulaire: ROUSSEL UCLAF, 93230 Romainville (FR)
(72) Inventeur: Buendia, Jean, F-94170 Le Perreux sur Marne (FR); Vivat, Michel, F-77400 Lagny sur Marne (FR)
(74) Mandataire: Vieillefosse, Jean-Claude

(56) Documents cités:
- EP-A- 0 058 097
- EP-A- 0 175 540
- EP-A- 0 336 521
- DE-A- 2 603 266
- LU-A- 68 212
- JOURNAL OF THE CHEMICAL SOCIETY. CHEMICAL COMMUNICATIONS. no. 15 , 1981 , LETCHWORTH GB pages 775 - 777 L. NEDELEC ET AL 'Simple Route for Elaboration of the Hydroxy-ketone and Dihydroxy-acetone Side-chains of Corticosteroids from 17-Oxo-steroids'
- CHEMICAL ABSTRACTS, vol. 54, no. 18, 25 Septembre 1960, Columbus, Ohio, US; abstract no. 18779g, A. ALIBRANDI ET AL 'Factors Influencing the Biological Activity of Orally Administered Steroid Compounds: Effect of the Medium and of Esterification' colonne 18779 ;
- JOURNAL OF MOLECULAR STRUCTURE vol. 99, no. 1-2 , 1983 , AMSTERDAM NL pages 133 - 136 O. EGYED ET AL 'Infrared Intensities of Pregna-1,4-diene-3,20-dione Derivatives'
- JOURNAL OF STEROID BIOCHEMISTRY AND MOLECULAR BIOLOGY vol. 43, no. 6 , 1992 , OXFORD GB pages 543 - 547 K. UNDISZ ET AL 'Bioconversion of Steroids by Cochliobolus lunatus. II. 11 -Hydroxylation of 17.alpha,21-Dihydroxy pregna-1,4-diene-3,20-dione 17-Acetate in Dependence of the Inducer Structure'

## Description

L'invention a pour objet un nouveau procédé de préparation de dérivés 20-oxo 17α, 21-dihydroxylés du pregnane et des nouveaux intermédiaires.

L'invention a ainsi pour objet un procédé de préparation des composés de formule (I) : 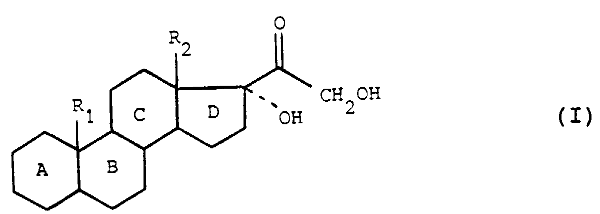 dans laquelle R₁ représente un atome d'hydrogène, un radical alkyle renfermant de 1 à 4 atomes de carbone, éventuellement substitué par une fonction oxygénée ou azotée ou par un atome d'halogène, ou R₁ représente un radical alkényle ou alkynyle renfermant de 2 à 4 atomes de carbone, R₂ représente un radical alkyle renfermant de 1 à 4 atomes de carbone, les noyaux A, B, C et D portent éventuellement une ou plusieurs doubles liaisons et sont éventuellement substitués par une ou plusieurs fonctions hydroxyle ou cétone éventuellement protégées, par un ou plusieurs atomes d'halogène, par un ou plusieurs radicaux alkyle ou alkyloxy renfermant de 1 à 4 atomes de carbone, ou par un ou plusieurs radicaux alkényle ou alkynyle renfermant de 2 à 4 atomes de carbone, caractérisé en ce que l'on traite un composé deformule (II) : 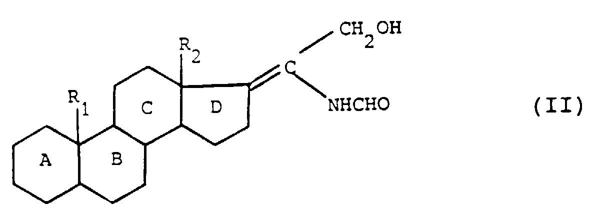 dans laquelle R₁, R₂, A, B, C et D sont définis comme précédemment, par un agent d'oxydation, en présence d'eau, et au sein d'un solvant au moins partiellement miscible à l'eau, pour obtenir le composé de formule (III) : 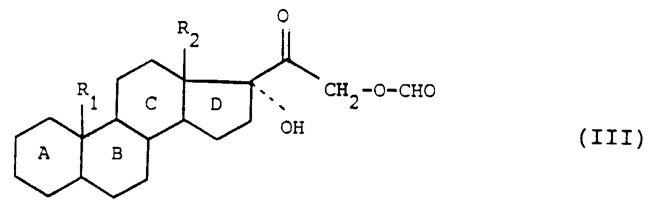 dans laquelle R₁, R₂, A, B, C et D sont définis comme précédemment, que l'on soumet à une solvolyse en milieu acide ou basique puis, le cas échéant, à une réaction de déprotection des fonctions hydroxyle et cétone protégées, éventuellement présentes.

Lorsque R₁ représente un radical alkyle, il s'agit de préférence du radical méthyle ou éthyle.

Lorsque R₁ représente un radical alkyle subsitué par une fonction oxygénée, il s'agit de préférence de radical hydroxyméthyle ou hydroxyéthyle, du radical formyle ou du radical acétyle.

Lorsque R₁ représente un radical alkyle substitué par une fonction azotée, il s'agit de préférence du radical cyano ou du radical aminométhyle ou aminoéthyle.

Lorsque R₁ représente un radical alkyle substitué par un halogène, il s'agit de préférence d'un radical -CH₂Hal, dans lequel Hal représente un atome de chlore, de fluor ou de brome.

Lorsque R₁ représente un radical alkényle, il s'agit de préférence du radical vinyle ou allyle.

Lorsque R₁ représente un radical alkynyle, il s'agit de préférence du radical éthynyle.

R₂ représente de préférence un radical méthyle ou éthyle.

Lorsque les noyaux A, B, C et D portent une ou plusieurs doubles liaisons, il s'agit de préférence de doubles liaisons en 1(2), 3(4), 4(5) ou 9(11) ou d'un système de doubles liaisons sons conjuguées en 3(4) et 5(6) ou en 4(5) et 6(7) ou en 1(2) et 4(5) ou d'un système aromatique de trois doubles liaisons 1,3,5(10) ou d'un système de trois doubles liaisons 1(2), 4(5), 6(7).

Lorsque les cycles, A, B, C et D sont substitués par une ou plusieurs fonctions hydroxyles, il s'agit de préférence d'une fonction hydroxyle en 3, en 9 ou en 11. Lorsque la ou les fonctions hydroxyles sont protégées, il s'agit de préférence d'une protection sous la forme d'esters d'acides organiques, par exemple de l'acide acétique ou formique, ou d'éthers d'alkyle inférieur, par exemple de méthyle ou d'éthyle, d'éthers silylés, par exemple de trialkylsilyle tel que triméthyl ou diméthyl-tert-butylsilyle, de triarylsilyle tel que triphénylsilyle ou de diarylalkylsilyle tel que diphényl tert-butylsilyle, ou encore d'éther de tétrahydropyranyle.

Lorsque les cycles A, B, C et D sont substitués par une ou plusieurs fonctions cétones, il s'agit de préférence d'une fonction cétone en 3 ou en 11. Lorsque la fonction cétone en 3 est protégée, il s'agit de préférence d'une protection sous la forme d'un cétal ou d'un thioacétal cyclique ou non, ou d'un éther d'énol ou encore d'une oxime. Lorsque la fonction cétone en 11 est protégée, il s'agit de préférence d'une protection sous la forme d'un éther d'énol.

Lorsque les cycles A, B, C et D sont substitués par un ou plusieurs atomes d'halogène, il s'agit de préférence d'un atome de fluor, de chlore ou de brome, en position 6 ou 9α.

Lorsque les cycles A, B, C et D sont substitués par un ou plusieurs radicaux alkyles, il s'agit de préférence du radical méthyle ou éthyle en 2, 6, 7, en 16α ou en 16β.

Lorsque les cycles A, B, C et D sont substitués par un ou plusieurs radicaux alkyloxy, il s'agit de préférence d'un radical méthoxy ou éthoxy en 3 ou 11β.

Lorsque les cycles A, B, C et D sont substitués par un ou plusieurs radicaux alkényles, il s'agit de préférence du radical vinyle ou allyle en position 11β par exemple.

Lorsque les cycles A, B, C et D sont substitués par un ou plusieurs radicaux alkynyles, il s'agit de préférence du radical éthynyle en position 11β par exemple.

L'invention a notamment pour objet un procédé tel que défini précédemment caractérisé en ce que l'on utilise au départ un composé de formule (II) répondant à la formule (II') : 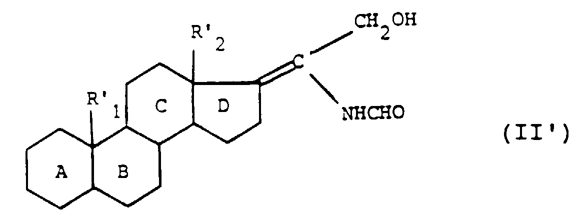 dans laquelle R'₁ représente un atome d'hydrogène ou un radical méthyle, R'₂ représente un radical méthyle ou éthyle, les noyaux A, B, C et D portent éventuellement une ou plusieurs doubles liaisons en position 1(2), 3(4), 4(5) ou 9(11) ou 3(4) et 5(6) ou 4(5) et 6(7) ou 1(2) et 4(5) ou 1,3,5(10) ou 1(2), 4(5) et 6(7) et sont éventuellement substitués par une ou plusieurs fonctions hydroxyle en 3, 9 et/ou 11, par une ou deux fonctions cétone en 3 et/ou 11, par un ou deux atomes de fluor, de chlore ou de brome en 6 et/ou 9α, par un ou plusieurs radicaux méthyle ou éthyle en 2, 6, 7 et/ou 16α ou 16β, par un ou deux radicaux méthoxy ou éthoxy en 3 et/ou 11β, par un radical vinyle ou allyle en 11β ou par un radical éthynyle en 11β.

L'invention a plus particulièrement pour objet un procédé tel que défini précédemment, caractérisé en ce que l'on utilise au départ un composé de formule (II) répondant à la formule (II") : 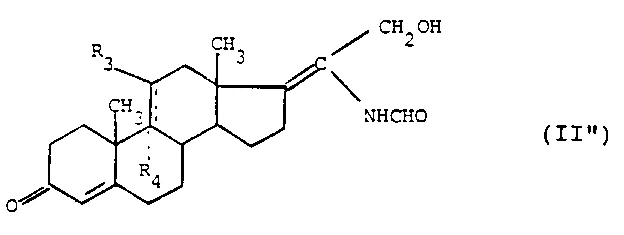 dans laquelle soit le trait pointillé en 9(11) représente une seconde liaison et R₃ représente un atome d'hydrogène, soit le trait pointillé ne représente pas une seconde liaison et R₃ représente un atome d'hydrogène, un radical β-hydroxy ou un radical oxo et R₄ représente un atome d'hydrogène, soit R₃ représente un atome d'hydrogène et R₄ représente un radical α-hydroxy.

L'invention a donc plus particulièrement pour objet un procédé tel que défini précédemment, caractérisé en ce que l'on utilise au départ :
- le composé de formule 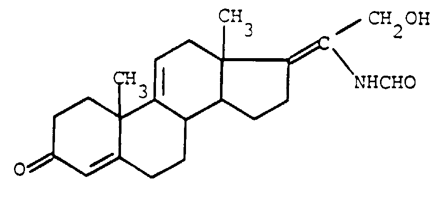
- le composé de formule 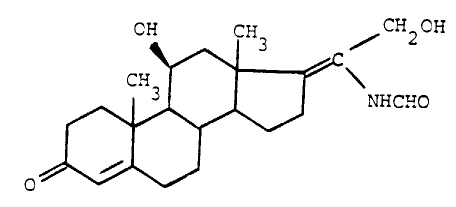
- le composé de formule 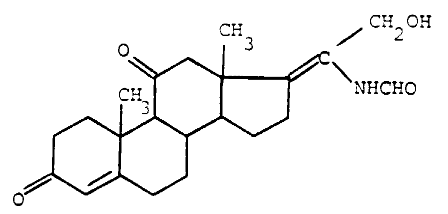
- le composé de formule 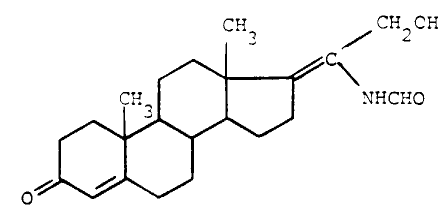 ou le composé de formule

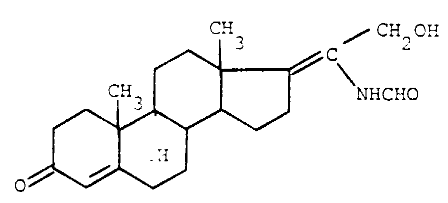

L'agent d'oxydation utilisé dans le procédé selon l'invention peut être choisi dans le groupe constitué par les peracides, par exemple l'acide perbenzoïque, métachloroperbenzoïque, peracétique, persuccinique, perphtalique, performique ou pertungstique, l'eau oxygénée utilisée seule ou en présence d'hexachloro ou hexafluoroacétone ou les hydroperoxydes, par exemple l'hydroperoxyde de tert-butyle, utilisé en présence d'acétylacétonate de vanadium en quantité catalytique. Les peracides, et tout spécialement l'acide perphtalique, sont particulièrement préférés.

Il va de soi que l'on peut, sans sortir du cadre de l'invention, utiliser un peracide préparé extemporanément par action de l'eau oxygénée sur l'acide ou l'anhydride correspondant.

Le solvant utilisé peut être un alcool, par exemple le méthanol, l'éthanol, l'isopropanol, un éther, par exemple le tétrahydrofuranne, le dioxanne, un ester, par exemple l'acétate d'éthyle, une cétone, par exemple l'acétone, la méthyléthylcétone, un amide, par exemple le diméthylformamide ; il peut encore être par exemple, l'acétonitrile ou l'acide acétique. Les alcanols sont particulièrement préférés.

On peut le cas échéant, opérer en présence d'un agent de transfert de phase, lequel peut être, par exemple, le chlorure de triéthylbenzylammonium ou le bromure de tétrabutylammonium. La solvolyse du composé de formule (III) est de préférence une alcoolyse effectuée en présence d'une base telle qu'un hydroxyde alcalin, par exemple la soude ou la potasse, ou un carbonate alcalin, par exemple le carbonate de sodium ou de potassium, ou en présence d'un acide fort aqueux, lequel peut être un acide minéral ou organique tel que l'acide chlorhydrique, bromhydrique, sulfurique, acétique.

La base peut être utilisée en quantité catalytique. L'alcool utilisé est, de préférence, le méthanol ou l'éthanol.

La solvolyse peut encore être une hydrolyse, effectuée en mettant en oeuvre les bases ci-dessus.

Ainsi qu'il est indiqué plus haut, on peut utiliser au départ du procédé de l'invention, un composé de formule (II) dans laquelle les fonctions hydroxyle et cétone éventuellement présentes sont protégées. Il va de soi que selon la nature du ou des groupements protecteurs, à savoir qu'ils sont ou ne sont pas sensibles en milieu acide ou basique, les composés de formule (III) puis (I) obtenus pourront comporter des fonctions déprotégées et que si l'on souhaite l'éviter, on choisira une protection appropriée. Ce choix est bien entendu à la portée de l'homme du métier.

L'invention a également pour objet un procédé tel que défini précédemment, caractérisé en ce que l'on opère sans isolement intermédiaire du composé de formule (III).

Les conditions préférées de mise en oeuvre sont les mêmes que celles qui ont été précisées plus haut.

On peut seulement indiquer qu'il peut être préférable de neutraliser le pouvoir oxydant du milieu avant la solvolyse du formiate de formule (III).

Les composés de formule (II) sont en général connus et décrits en particulier dans le brevet européen 023 856. Certains de ces composés comportant des fonctions hydroxy ou cétone peuvent n'avoir été décrits que sous une forme protégée ou que sous la forme déprotégée. Il est bien entendu à la portée de l'homme du métier de préparer une forme ou une autre à partir du composé connu.

L'oxydation des ène-amides de formule (II) par des peracides a déjà fait l'objet d'essais dans le passé, dans le but de préparer des composés de type (I). On se reportera en particulier à la publication Nédelec et coll. J. Chem. Soc. Chem. Comm. (1981) p. 775. Il y est indiqué que les essais sur un composé comportant un cycle A aromatique ont été infructueux, car ils ont conduit à une coupure de la chaîne en 17, conduisant au dérivé 17-oxo correspondant. Un autre essai de ce type appliqué à une pregna 4, 17(20)-dièn-3-one a été décrit ci-après dans la partie expérimentale.

Selon la publication ci-dessus, l'introduction d'une fonction oxygénée sur la chaîne en 17 avait été seulement possible en utilisant un réactif particulier, le tétracétate de plomb, selon une méthode décrite par Barton et coll. J. Chem. Soc. Perkin. Trans. 1, (1975), 1242. Cette méthode conduisait intermédiairement à un diacétate en 17α,21. C'est cette méthode particulière qui a été également utilisée pour oxyder un dérivé 9α-OH dans la demande de brevet européen 0 336 521 et il y est indiqué que le réactif est utilisé préférentiellement en milieu anhydre.

Il s'avère en fait que l'enchaînement particulier énamide en position 17 est extrêmement fragile vis à vis des oxydants et en particulier des peracides et il a été admis à la suite de la publication de Nédelec et coll. que ces ènamides qui constituent des intermédiaires de choix dans la reconstitution de la chaîne en 17 des cortisoniques nécessitaient l'utilisation d'un réactif particulier, lequel est avant tout un réactif d'hydroxylation et non d'oxydation.

Le procédé de la présente demande a permis de vaincre ce préjugé en démontrant qu'il est possible dans des conditions non évidentes dans ce type de réaction, à savoir en présence d'eau, d'oxyder avec de bons rendements les énamides de formule (II), en particulier par des peracides, donc dans des conditions industrielles, pour obtenir les dérivés 17α,21-dihydroxy correspondants. Le procédé conduit intermédiairement à un monoformiate en 21 [composé (III)] qu'il est très facile d'hydrolyser, en particulier par une alcoolyse en milieu basique ou acide.

L'invention a enfin pour objet, à titre de composés industriels nouveaux et notamment à titre d'intermédiaires nécessaires à la mise en oeuvre du procédé selon l'invention, les composés de formule (III′) : 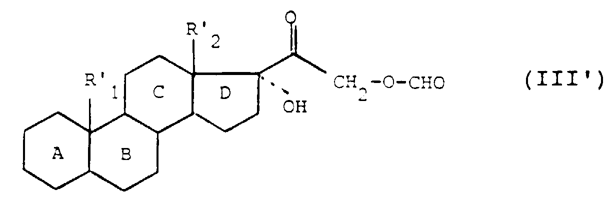 dans laquelle R'₁ représente un atome d'hydrogène ou un radical méthyle, R'₂ représente un radical méthyle ou éthyle, les noyaux A, B, C et D portent éventuellement une ou plusieurs doubles liaisons en position 1(2), 3(4), 4(5) ou 9(11) ou 3(4) et 5(6) ou 4(5) et 6(7) ou 1(2) et 4(5) ou 1,3,5(10) ou 1(2), 4(5) et 6(7) et sont éventuellement substitués par une ou plusieurs fonctions hydroxyle en 3, 9 et/ou 11, par une ou deux fonctions cétone en 3 et/ou 11, par un ou deux atomes de fluor, de chlore ou de brome en 6 et/ou 9alpha, par un ou plusieurs radicaux méthyle ou éthyle en 2, 6, 7 et/ou 16alpha ou 16béta, par un ou deux radicaux méthoxy ou éthoxy en 3 et/ou 11béta, par un radical vinyle ou allyle en 11béta ou par un radical éthynyle en 11béta, à l'exception du 21-formiate de bêta methasone et des composés dans lesquels R'₂ représente un radical méthyle et soit R'₁ représente un radical méthyle, deux doubles liaisons sont présentes en position 1 et 4, un radical oxo en 3, un radical hydroxy ou oxo en 11 ou un radical hydroxy en 11 et un radical méthyle en 16béta, soit R'₁ représente un radical méthyle, une double liaison est présente en position 4, une fonction céto en 3 et un atome d'hydrogène, un radical hydroxy ou un radical oxo en 11, soit R'₁ représente un radical méthyle, une fonction hydroxy est présente en 3 et une fonction oxo en 11, soit R'₁ représente un atome d'hydrogène une double liaison est présente en 4 et une fonction céto en 3 et notamment le composé de formule : 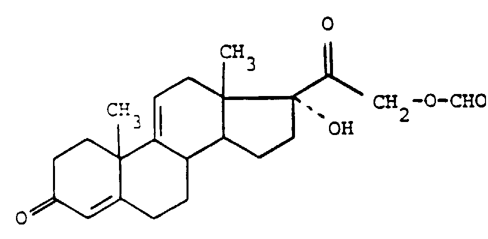 et le composé de formule

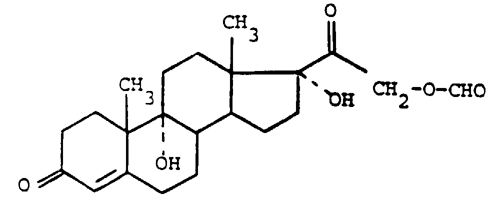

Les composés de formule (I) obtenus selon l'invention sont soit des composés connus pour leurs propriétés thérapeutiques, soit des intermédiaires connus pouvant être utilisés dans la préparation de composés thérapeutiquement actifs.

Les exemples suivants illustrent l'invention sans toutefois la limiter.

### Exemple 1: 11β,17α,21-trihydroxy pregn-4-ène-3,20-dione (Hydrocortisone).

### Stade A : 21-formyloxy 11β,17α-dihydroxy pregn-4-ène-3,20-dione.

On mélange sous gaz inerte 0,81 g de 20-formamido 11β,21-dihydroxy pregna-4,17(20)-dièn-3-one, 6,4 cm³ d'eau et 6,4 cm³ de méthanol. On ajoute sous agitation à 22-23°C, 0,8 cm³ d'acide perphtalique à 50% (obtenus à partir de 2 g d'anhydre phtalique que l'on solubilise à 40°C en 15 minutes dans un mélange de 1 cm³ d'eau oxygénée à 50% et 2 cm³ de méthanol), puis après 1 heure 30 minutes sous agitation, à nouveau 0,8 cm³ d'acide perphtalique et, après 4 heures, à nouveau 0,5 cm³ d'acide perphtalique. Après 3 heures sous agitation, on refroidit à 5°C et essore les cristaux puis les lave à l'eau et par une solution aqueuse de bicarbonate de sodium et les sèche. On obtient 0,586 g de produit attendu.

### Spectre IR (nujol) :

Absorptions à 3420 et 3315 cm⁻¹ (OH), 1735, 1715 et 1625 cm⁻¹ (C=O formiate, 20-céto et delta 4-3-céto).

### Spectre RMN (DMSO) :

18-CH₃ : 0,77 (s) ; 19-CH₃ : 1,37 (s) ; H₁₁ : 4,27 (m) ; 1H (mobile) : 4,36 (d) et 5,44 (s) ; CO-CH₂-O : 4,83 (d) et 5,18 (d) ; H₄ : 5,56 (s) ; -CHO : 8,33 (s).

### Stade B : 11β,17α,21-trihydroxy pregn-4-ène-3,20-dione (Hydrocortisone).

On mélange sous gaz inerte 0,575 g de produit obtenu au stade A et 10 cm³ de méthanol puis ajoute 0,05 g de carbonate de potassium. On maintient sous agitation pendant 30 minutes puis ajoute 0,7 cm³ d'acide sulfurique 1N. On concentre sous pression réduite et chromatographie le résidu sur silice en éluant par un mélange chloroforme-méthanol (95-5). On obtient ainsi 0,502 g d'hydrocortisone. F = 210°C.
[α]_{D} = +151,6° (c = 1% EtOH).

### Exemple 2 : Essai en milieu anhydre.

On mélange sous gaz inerte 0,05 g de 20-formamido 11β,21-dihydroxy pregna 4,17(20)-dièn-3-one et 0,5 cm³ de méthanol. On ajoute à température ambiante 0,05 g d'acide perphtalique et maintient sous agitation pendant 20 heures. L'analyse chromatographique du milieu réactionnel dans le système solvant chlorure de méthylène-méthanol (95-5) révèle l'absence de produit de départ, et l'absence totale de 21-formiate d'hydrocortisone et la présence en quantité importante de 11β-hydroxy androst-4-èn 3,17-dione, identifiée par comparaison avec un échantillon de référence Rf = 0,39.

### Spectre RMN (CDCl₃, 300 MHz) :

1,17 (s) : 18-CH₃ ; 1,47 (s) : 19-CH₃ ; 4,47 (q) : H₁₁ équatorial ; 5,70 (d) : H₄.

### Préparation : 20-formamido 11β,21-dihydroxy pregna-4,17(20)-dièn-3-one.

On dissout 9 g de 3-éthoxy 11β,21-dihydroxy 20-formamido pregna-3,5,17(20)-triène (décrit dans le brevet européen 023 856) dans 90 cm³ d'acide acétique à 5% d'eau et agite pendant 30 minutes à température ambiante, sous gaz inerte. On refroidit à 0°C environ et ajoute lentement 140 cm³ d'ammoniaque à 22°Bé et extrait au chloroforme. On lave la phase organique à l'eau, la sèche et l'amène à sec. On obtient 8,1 g de produit attendu que l'on peut purifier par chromatographie sur silice en éluant au mélange chloroforme-méthanol (95-5). F ≈ 238°C.
[α]_{D} = +91° ± 2,5° (c = 0,5% éthanol).

### Spectre IR (nujol) :

absorptions à 3435-3242 cm⁻¹ (NH/OH) ; 1665-1655 cm⁻¹ (C=O) ; 1610-1523 cm⁻¹ (C=C conjuguée-NH déformation).

### Exemple 3 : 17α,21-dihydroxy pregn-4-ène-3,20-dione.

### Stade A : 21-formyloxy 17α-hydroxy pregn-4-ène-3,20-dione.

On agite à température ambiante 2 g de 20-formamido 21-hydroxy pregna 4,17(20)-dièn-3-one, dans 20 cm³ d'eau et 20cm³ de méthanol. On introduit lentement en 3 heures et demi, à la température de 23°C, 7 cm³ de peracide obtenu à partir de 2,66 g d'anhydride perphtalique, 4 cm³ de méthanol et 1,9 cm³ d'eau oxygénée à 50% et 2 heures d'agitation. On maintient sous agitation le milieu réactionnel puis ajoute en 1 heure, 20 cm³ d'eau, agite la suspension obtenue, essore, lave avec un mélange méthanol-eau (1-1), sèche sous pression réduite et recueille 1,4 g de produit brut que l'on chromatographie sur silice (éluant : chlorure de méthylène-méthanol 85-15 puis 80-20). On obtient 0,9 g de produit attendu. F = 186°C.

### Spectre IR :

absorptions à 3615 cm⁻¹ OH (+ associé) ; 1739 cm⁻¹ 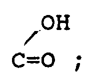 1722 cm⁻¹ 20-céto ; 1661 cm⁻¹ 3-céto Δ4 ; 1615 cm⁻¹ C=C.

### Stade B : 17α,21-dihydroxy pregn-4-ène-3,20-dione.

On mélange pendant 30 minutes sous atmosphère inerte 0,27 g de produit préparé au stade A dans 5 cm³ de méthanol, ajoute 20 mg de carbonate de potassium, agite 30 minutes, ajoute 10 cm³ d'eau, agite 10 minutes, essore le précipité, le lave à l'eau, le sèche sous pression réduite et recueille 0,204 g de produit attendu.

### Spectre IR (CHCl₃) :

Absorption à 3615 cm⁻¹ (OH) ; 1708-1661 cm⁻¹ (C=O) ; 1615 cm⁻¹ (C=C).

### Préparation du 20-formamido 21-hydroxy pregna 4,17(20)-dièn-3-one.

### Stade A : 3-éthoxy 3,5-androstadièn-17-one.

On agite à 65°C sous atmosphère inerte 50 g d'androst-4-ène-3,17-dione dans 150 cm³ d'éthanol en présence de 50 cm³ d'orthoformiate d'éthyle. Après complète solubilisation, on ajoute 2,5 cm³ d'une slution éthanolique d'acide sulfurique (0,2 cm³/100 cm³). Après 1 heure de réaction en laissant revenir à 60°C puis à 50°C, on amorce la cristallisation, refroidit à 25°C en 1 heure et demie, ajoute 10 cm³ d'eau, agite 1 heure et demie, ajoute de nouveau 10 cm³ d'eau, agite 40 minutes, essore le précipité formé, le lave avec un mélange eau-alcool 25-75 puis 50-50 et le sèche sous pression réduite. On obtient 46,5 g de produit attendu.

### Spectre IR (CHCl₃) :

Absorption à 1732 cm⁻¹(17-céto);1652-1626 cm⁻¹ 3(EtO)3,5-diène

### Stade B : 3-éthoxy 20-formamido pregna 3,5,17(20)-trièn-21-oate d'éthyle.

On dissout 7 g de terbutylate de potassium dans 38 cm³ de tétrahydrofuranne, refroidit à 0°/+5°C, ajoute en 20 minutes 6,8 cm³ d'isocyanoacétate d'éthyle en solution dans 38 cm³ de tétrahydrofuranne, agite 15 minutes, ajoute en 30 minutes 15 g du produit préparé au stade A dans 75 cm³ de tétrahydrofuranne. On maintient 4 heures entre 0°/+5°C, ajoute une solution aqueuse de chlorure d'ammonium (7,5 g/75 cm³), concentre partiellement sous pression réduite à température ambiante. On ajoute 80 cm³ d'eau à la suspension formée, essore le précipité, le lave à l'eau, le sèche sous pression réduite à 35°C et obtient 20,7 g de produit attendu.

### Spectre IR (CHCl₃) :

Absorptions à 3415 et 3390 cm⁻¹ (NH) ; 1695 cm⁻¹ (C=O) ; 1652 et 1626 cm⁻¹ (C=C).

### Stade C : 20-formamido 21-hydroxy pregna 4,17(20)dièn-3-one.

On refroidit à -5°C, sous atmosphère inerte 10 cm³ d'hydrure d'aluminium-lithium dans 100 cm³ de tétrahydrofuranne, ajoute 10 g de produit obtenu au stade B puis agite 1 heure et demie à 0°C. On ajoute 2 g de chlorure d'ammonium puis en 50 minutes en laissant la température revenir vers +10°C, on ajoute 40 cm³ d'une solution à 25% de chlorure d'ammonium. On agite 15 minutes, essore le précipité, le lave avec un mélange chlorure de méthylène-méthanol (1-1), évapore le solvant et obtient 10 g de produit brut que l'on reprend dans 50 cm³ de chloroforme, ajoute 5 cm³ d'eau et 5 cm³ d'acide acétique, agite 1 heure, ajoute de nouveau 5 cm³ d'acide acétique et maintient 5 heures sous agitation. On neutralise par addition de soude 2N, décante, extrait la phase aqueuse au chlorure de méthylène, sèche et élimine le solvant sous pression réduite. Après chromatographie sur silice (éluant : chlorure de méthylène-isopropanol 3% à 12%), on obtient 2,75 g de produit attendu.

### Spectre IR (CHCl₃) :

Absorption à 3610 cm⁻¹ (OH) ; 3440 cm⁻¹ (NH) ; 1672 cm⁻¹ (cétone conjuguée + formyle) ; 1616 cm⁻¹ (C=C).

### Exemple 4 : 17α,21-dihydroxy pregna 4,9(11)-diène-3,20-dione.

### Stade A : 21-formyloxy 17α-hydroxy pregna 4,9(11)-diène-3,20-dione.

On agite à température ambiante 0,4 g de 20-formamido 21-hydroxy pregna 4,9(11),17(20)-trièn-3-one, dans 4 cm³ d'eau et 2 cm³ de méthanol. On introduit lentement en 4 heures et demie, à la température de 23°C, 1,2 cm³ de peracide obtenu à partir de 0,532 g d'anhydride perphtalique, 0,8 cm³ de méthanol et 0,4 cm³ d'eau oxygénée à 50% et agitation à 45°C. On maintient sous agitation le milieu réactionnel puis ajoute en 1 heure, 4 cm³ d'eau, agite 30 minutes la suspension obtenue, essore, lave avec un mélange méthanol-eau (75-25), sèche sous pression réduite et recueille 0,18 g de produit brut comprenant un mélange de produit attendu et d'époxyde α9,11 correspondant.

### Spectre IR :

à absorptions à 3614 cm⁻¹OH (+ associé) ; 1740 cm⁻¹ ; 1722 cm⁻¹ 20-céto ; 1666 cm⁻¹ 3-céto Δ4 ; 1616 cm⁻¹ C=C.

### Stade B : 17α,21-dihydroxy pregna 4,9(11)-diène-3,20-dione.

On mélange pendant 30 minutes sous atmosphère inerte 100 mg de mélange préparé au stade A dans 1,9 cm³ de méthanol, ajoute 7,2 mg de carbonate de potassium, agite 30 minutes, ajoute 5 cm³ d'eau, agite 15 minutes, essore le précipité, le lave à l'eau, le sèche sous pression réduite. Après chromatographie sur silice (éluant : chloroforme-isopropanol 95-5), on obtient 43 mg de produit attendu et 35 mg de l'époxyde α9 (10) correspondant.

### Spectre IR (CHCl₃) du cortinène :

Absorption à 3520-3480 cm⁻¹ (OH) ; 1710-1660 cm⁻¹ (C=O) ; 1610 cm⁻¹ (C=C).

### Préparation du 20-formamido 21-hydroxy pregna 4,9(11),17(20)-trièn-3-one.

### Stade A : 3-éthoxy 3,5,9(11)-androstatrièn-17-one.

On opère comme au stade A de la préparation de l'exemple 3 en utilisant au départ 50 g d'androst 4,9(11)-diène-3,17-dione. On obtient 37,4 g de produit attendu.

### Spectre IR (CHCl₃) :

### Absorption à 1732 cm⁻¹ (17-céto) ; 1656-1629 cm⁻¹ (3(EtO)3,5-diène.

### Stade B : 3-éthoxy 20-formamido pregna 3,5,9(11),17(20)-tétraène-21-oate d'éthyle.

On opère comme au stade B de la préparation de l'exemple 3 en utilisant 15 g de produit préparé au stade A précédent et obtient 20,1 g de produit attendu.

### Spectre IR (CHCl₃) :

Absorptions à 3417 et 3389 cm⁻¹ (NH) ; 1697 cm⁻¹ (C=O) ; 1656 et 1629 cm⁻¹ (C=C).

### Stade C : 3-éthoxy 20-formamido 21-hydroxy pregna 3,5,9(11),17(20)-tétraène.

On ajoute en 30 minutes 2,5 g de l'énamide préparé au stade B à 2,8 cm³ d'hydrure d'aluminium -lithium dans 25 cm³ de tétrahydrofuranne refroidi à 0°/-5°C. On agite 15 minutes, ajoute en 45 minutes 1,6 cm³ de phosphate monosodique en solution aqueuse (à 30%), ajoute 10 cm³ de tétrahydrofuranne et agite la suspension pendant 1 heure et demie en laissant revenir à température ambiante. On filtre, lave avec un mélange tétrahydrofuranne-chloroforme 1-1 puis chloroformeéthanol 2-1, évapore les solvants du filtrat, chromatographie le résidu sur silice (éluant : chlorure de méthylèneisopropanol 92,5-7,5 à 2°/ₒₒ de triéthylamine) et recueille 1,49 g de produit attendu.

### Spectre IR (CHCl₃) :

Absorptions à 3437 cm⁻¹ (NH) ; 1608 cm⁻¹ (C=O) ; 3612 cm⁻¹ (OH) ; 1657 et 1629 cm⁻¹ 3(EtO)3,5-diène.

### Stade D : 20-formamido 21-hydroxy pregna 4,9(11),17(20)-trièn-3-one.

On agite pendant 3 heures et demie sous atmosphère inerte, 1 g du produit obtenu au stade C dans 5 cm³ d'eau additionné de 5 cm³ d'acide acétique à 99,5%. On verse sur un mélange eau/glace, ajoute 2,9 g de bicarbonate de sodium, extrait la phase aqueuse au chlorure de méthylène, sèche, évapore le solvant, chromatographie le résidu sur silice (éluant : chlorure de méthylène-isopropanol 92-8) et récupère 0,55 g de produit attendu.

## Revendications

1. Procédé de préparation des composés de formule (I) : 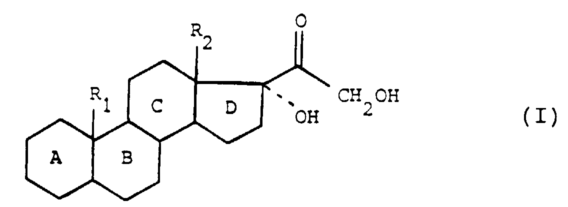 dans laquelle R₁ représente un atome d'hydrogène, un radical alkyle renfermant de 1 à 4 atomes de carbone, éventuellement substitué par une fonction oxygénée ou azotée ou par un atome d'halogène, ou R₁ représente un radical alkényle ou alkynyle renfermant de 2 à 4 atomes de carbone, R₂ représente un radical alkyle renfermant de 1 à 4 atomes de carbone, les noyaux A, B, C et D portent éventuellement une ou plusieurs doubles liaisons et sont éventuellement substitués par une ou plusieurs fonctions hydroxyle ou cétone éventuellement protégées, par un ou plusieurs atomes d'halogène, par un ou plusieurs radicaux alkyle ou alkyloxy renfermant de 1 à 4 atomes de carbone, ou par un ou plusieurs radicaux alkényle ou alkynyle renfermant de 2 à 4 atomes de carbone, caractérisé en ce que l'on traite un composé deformule (II) : 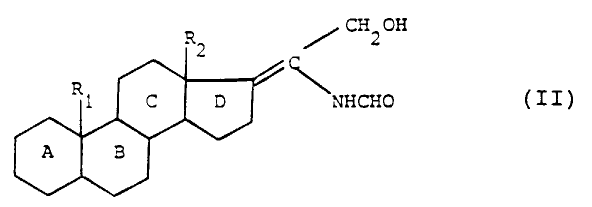 dans laquelle R₁, R₂, A, B, C et D sont définis comme précédemment, par un agent d'oxydation, en présence d'eau, et au sein d'un solvant au moins partiellement miscible à l'eau, pour obtenir le composé de formule (III) : 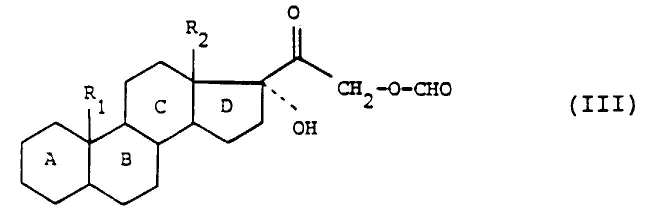 dans laquelle R₁, R₂, A, B, C et D sont définis comme précédemment, que l'on soumet à une solvolyse en milieu acide ou basique puis, le cas échéant, à une réaction de déprotection des fonctions hydroxyle et cétone protégées, éventuellement présentes.

2. Procédé selon la revendication 1, caractérisé en ce que l'on utilise au départ un composé de formule (II) répondant à la formule (II') : 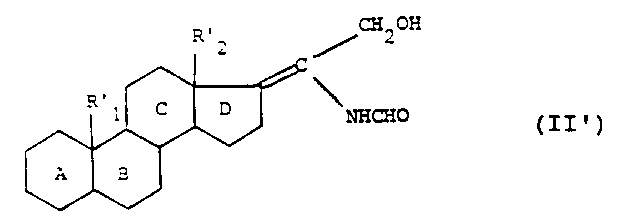 dans laquelle R'₁ représente un atome d'hydrogène ou un radical méthyle, R'₂ représente un radical méthyle ou éthyle, les noyaux A, B, C et D portent éventuellement une ou plusieurs doubles liaisons en position 1(2), 3(4), 4(5) ou 9(11) ou 3(4) et 5(6) ou 4(5) et 6(7) ou 1(2) et 4(5) ou 1,3,5(10) ou 1(2), 4(5) et 6(7) et sont éventuellement substitués par une ou plusieurs fonctions hydroxyle en 3, 9 et/ou 11, par une ou deux fonctions cétone en 3 et/ou 11, par un ou deux atomes de fluor, de chlore ou de brome en 6 et/ou 9α, par un ou plusieurs radicaux méthyle ou éthyle en 2, 6, 7 et/ou 16α ou 16β, par un ou deux radicaux méthoxy ou éthoxy en 3 et/ou 11β, par un radical vinyle ou allyle en 11β ou par un radical éthynyle en 11β.

3. Procédé selon la revendication 1, caractérisé en ce que l'on utilise au départ un composé de formule (II) répondant à la formule (II") : 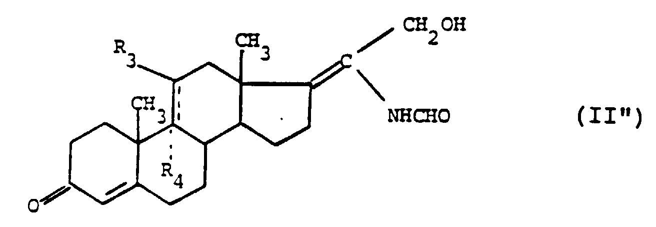 dans laquelle soit le trait pointillé en 9(11) représente une seconde liaison et R₃ représente un atome d'hydrogène, soit le trait pointillé ne représente pas une seconde liaison et R₃ représente un atome d'hydrogène, un radical β-hydroxy ou un radical oxo et R₄ représente un atome d'hydrogène, soit R₃ représente un atome d'hydrogène et R₄ représente un radical α-hydroxy.

4. Procédé selon l'une quelconque des revendications 1 3, caractérisé en ce que l'on utilise au départ le composé de formule

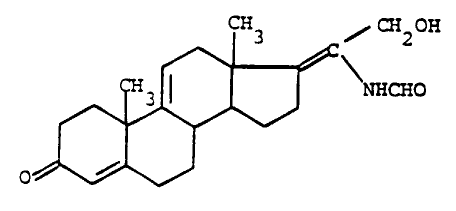

5. Procédé selon l'une quelconque des revendications 1 à 3, caractérisé en ce que l'on utilise au départ le composé de formule

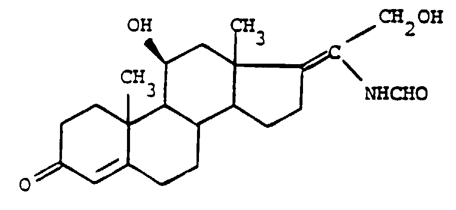

6. Procédé selon l'une quelconque des revendications 1 à 3, caractérisé en ce que l'on utilise au départ le composé de formule

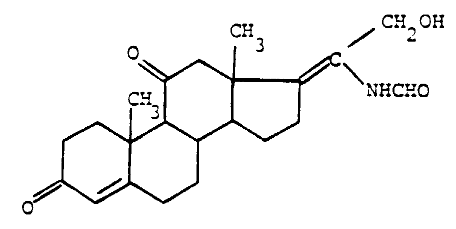

7. Procédé selon l'une quelconque des revendications 1 à 3, caractérisé en ce que l'on utilise au départ le composé de formule

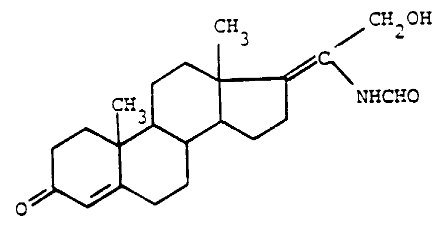

8. Procédé selon l'une quelconque des revendications 1 à 3, caractérisé en ce que l'on utilise au départ le composé de formule

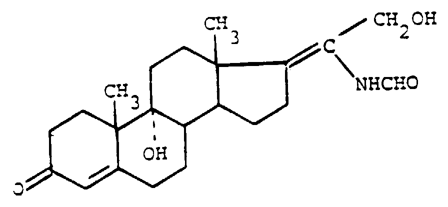

9. Procédé selon l'une quelconque des revendications 1 à 8, caractérisé en ce que l'agent d'oxydation est choisi dans le groupe constitué par les peracides, l'eau oxygénée et les hydroperoxydes.

10. Procédé selon la revendication 9, caractérisé en ce que l'agent d'oxydation est un peracide.

11. Procédé selon la revendication 9 ou 10, caractérisé en ce que l'agent d'oxydation est l'acide perphtalique.

12. Procédé selon l'une quelconque des revendications 1 à 11, caractérisé en ce que l'on opère au sein d'un alcanol.

13. Procédé selon l'une quelconque des revendications 1 à 12, caractérisé en ce que la solvolyse du composé de formule (III) est une alcoolyse effectuée en présence d'un hydroxyde ou d'un carbonate alcalin.

14. Procédé selon l'une quelconque des revendications 1 à 12, caractérisé en ce que la solvolyse du composé de formule (III) est une alcoolyse effectuée en présence d'un acide fort aqueux.

15. Procédé selon l'une quelconque des revendications 1 à 14, caractérisé en ce que l'on opère sans isolement intermédiaire du composé de formule (III).

16. A titre de composés industriels, les composés de formule (III') : 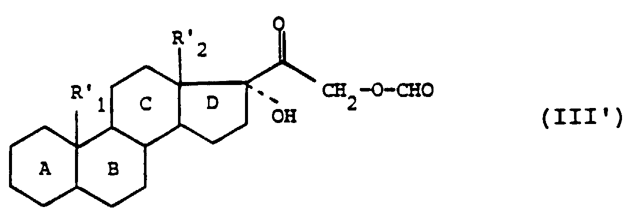 dans laquelle R'₁ représente un atome d'hydrogène ou un radical méthyle, R'₂ représente un radical méthyle ou éthyle, les noyaux A, B, C et D portent éventuellement une ou plusieurs doubles liaisons en position 1(2), 3(4), 4(5) ou 9(11) ou 3(4) et 5(6) ou 4(5) et 6(7) ou 1(2) et 4(5) ou 1,3,5(10) ou 1(2), 4(5) et 6(7) et sont éventuellement substitués par une ou plusieurs fonctions hydroxyle en 3, 9 et/ou 11, par une ou deux fonctions cétone en 3 et/ou 11, par un ou deux atomes de fluor, de chlore ou de brome en 6 et/ou 9alpha, par un ou plusieurs radicaux méthyle ou éthyle en 2, 6, 7 et/ou 16alpha ou 16béta, par un ou deux radicaux méthoxy ou éthoxy en 3 et/ou 11béta, par un radical vinyle ou allyle en 11béta ou par un radical éthynyle en 11béta, à l'exception du 21-formiate de bêta-méthasone et des composés dans lesquels R'₂ représente un radical méthyle et soit R'₁ représente un radical méthyle, deux doubles liaisons sont présentes en position 1 et 4, un radical oxo en 3, un radical hydroxy ou oxo en 11 ou un radical hydroxy en 11 et un radical méthyle en 16béta, soit R'₁ représente un radical méthyle, une double liaison est présente en position 4, une fonction céto en 3 et un atome d'hydrogène, un radical hydroxy ou un radical oxo en 11, soit R'₁ représente un radical méthyle, une fonction hydroxy est présente en 3 et une fonction oxo en 11, soit R'₁ représente un atome d'hydrogène une double liaison est présente en 4 et une fonction céto en 3.

17. A titre de composé industriel, selon la revendication 16, le composé de formule :

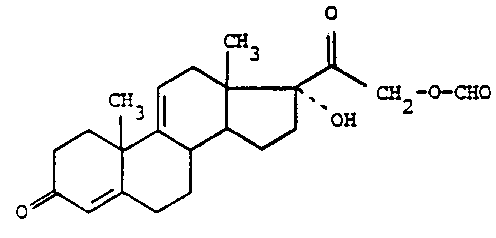

18. A titre de composé industriel, selon la revendication 16, le composé de formule

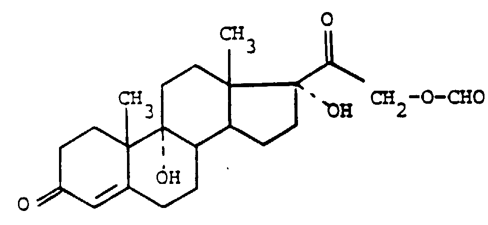

## Patentansprüche

1. Verfahren zur Herstellung der Verbindungen der Formel (I) 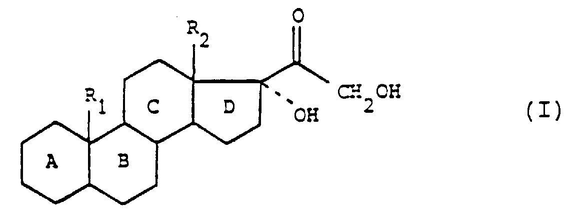 worin R₁ für ein Wasserstoffatom, einen Alkylrest mit 1 bis 4 Kohlenstoffatomen, gegebenenfalls substituiert durch eine Sauerstoff- oder Stickstoffunktion oder durch ein Halogenatom, steht oder R₁ für einen Alkenyl-oder Alkinylrest mit 2 bis 4 Kohlenstoffatomen steht, R₂ einen Alkylrest mit 1 bis 4 Kohlenstoffatomen bedeutet, die Ringe A, B, C und D gegebenenfalls eine oder mehrere Doppelbindungen aufweisen und gegebenenfalls durch eine oder mehrere gegebenenfalls geschützte Hydroxyl- oder Ketonfunktionen, durch ein oder mehrere Halogenatome, durch einen oder mehrere Alkyl- oder Alkoxyreste mit 1 bis 4 Kohlenstoffatomen oder durch einen oder mehrere Alkenyl- oder Alkinylreste mit 2 bis 4 Kohlenstoffatomen substituiert sind,
dadurch gekennzeichnet, daß man eine Verbindung der Formel (II) 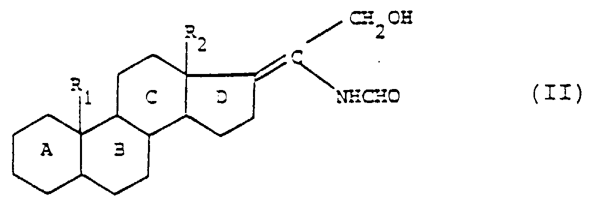 worin R₁, R₂, A, B, C und D wie vorstehend definiert sind, mit einem Oxidationsmittel in Anwesenheit von Wasser und in einem Lösungsmittel, welches zumindest teilweise mit Wasser mischbar ist, behandelt, um zu der Verbindung der Formel (III) 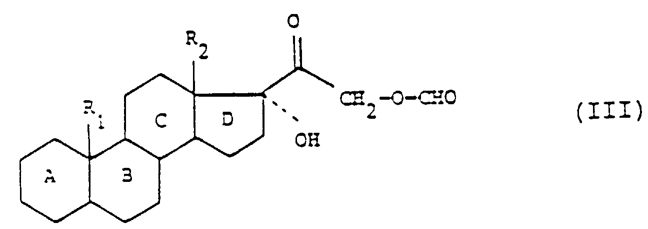 zu gelangen, worin R₁, R₂, A, B, C und D wie vorstehend definiert sind, welche man einer Solvolyse in saurem oder basischem Milieu und danach gegebenenfalls einer Reaktion zur Abspaltung der Schutzgruppen der gegebenenfalls vorhandenen geschützten Hydroxyl- und Ketonfunktionen unterzieht.

2. Verfahren gemäß Anspruch 1, dadurch gekennzeichnet, daß man von einer Verbindung der Formel (II), die der Formel (II') 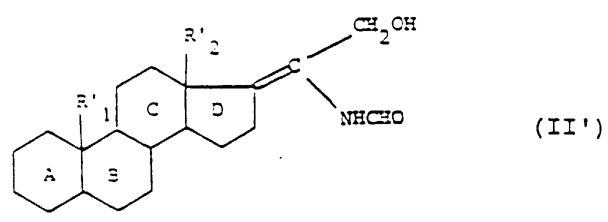 entspricht, ausgeht, worin R'₁ ein Wasserstoffatom oder einen Methylrest bedeutet, R'₂ für einen Methyl- oder Ethylrest steht, die Ringe A, B, C und D gegebenenfalls eine oder mehrere Doppelbindungen in 1(2)-, 3(4)-, 4(5)- oder 9(11)-oder 3(4)- und 5(6)- oder 4(5)- und 6(7)- oder 1(2)- und 4(5)-oder 1,3,5(10)- oder 1(2)-, 4(5)- und 6(7)-Stellung aufweisen und gegebenenfalls durch eine oder mehrere Hydroxylfunktionen in 3-, 9- und/oder 11-Stellung, durch eine oder zwei Ketonfunktionen in 3- und/oder 11-Stellung, durch ein oder zwei Fluor-, Chlor- oder Bromatome in 6- und/oder 9α-Stellung, durch einen oder mehrere Methyl- oder Ethylreste in 2-, 6-, 7- und/oder 16a- oder 16β-Stellung, durch einen oder zwei Methoxy- oder Ethoxyreste in 3- und/oder 11β-Stellung, durch einen Vinyl-oder Allylrest in 11β-Stellung oder durch einen Ethinylrest in 11β-Stellung substituiert sind.

3. Verfahren gemäß Anspruch 1, dadurch gekennzeichnet, daß man von einer Verbindung der Formel (II), die der Formel (II") 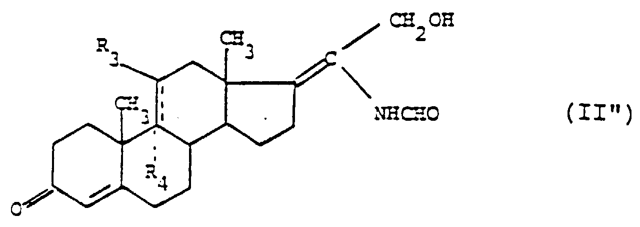 entspricht, ausgeht, worin entweder die gestrichelte Linie in 9(11)-Stellung für eine zweite Bindung steht und R₃ ein Wasserstoffatom bedeutet oder die gestrichelte Linie keine zweite Bindung darstellt und R₃ für ein Wasserstoffatom, einen β-Hydroxyrest oder einen Oxorest steht und R₄ ein Wasserstoffatom bedeutet oder R₃ für ein Wasserstoffatom steht und R₄ einen α-Hydroxyrest bedeutet.

4. Verfahren gemäß einem der Ansprüche 1 bis 3, dadurch gekennzeichnet, daß man von einer Verbindung der Formel 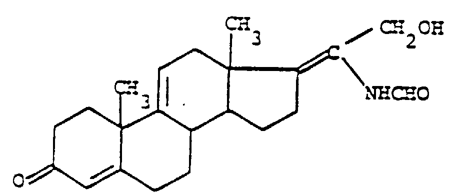 ausgeht.

5. Verfahren gemäß einem der Ansprüche 1 bis 3, dadurch gekennzeichnet, daß man von einer Verbindung der Formel 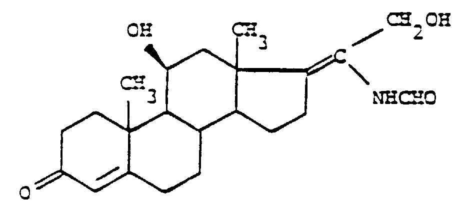 ausgeht.

6. Verfahren gemäß einem der Ansprüche 1 bis 3, dadurch gekennzeichnet, daß man von einer Verbindung der Formel 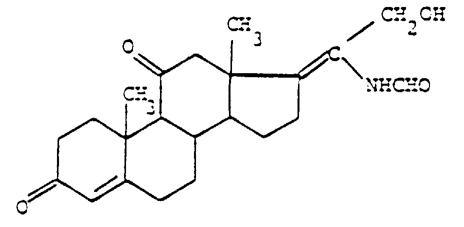 ausgeht.

7. Verfahren gemäß einem der Ansprüche 1 bis 3, dadurch gekennzeichnet, daß man von einer Verbindung der Formel 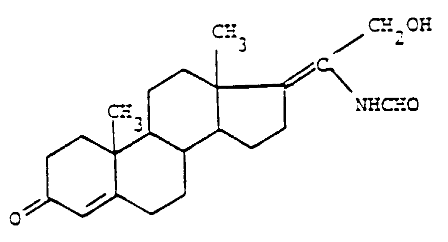 ausgeht.

8. Verfahren gemäß einem der Ansprüche 1 bis 3, dadurch gekennzeichnet, daß man von einer Verbindung der Formel 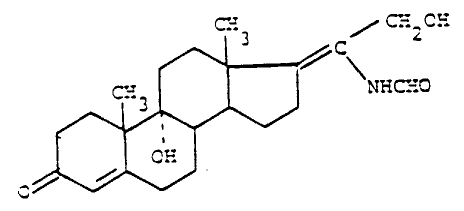 ausgeht.

9. Verfahren gemäß einem der Ansprüche 1 bis 8, dadurch gekennzeichnet, daß das Oxidationsmittel unter der Gruppe der Persäuren, des Wasserstoffperoxids und der Hydroperoxide ausgewählt wird.

10. Verfahren gemäß Anspruch 9, dadurch gekennzeichnet, daß das Oxidationsmittel eine Persäure ist.

11. Verfahren gemäß Anspruch 9 oder 10, dadurch gekennzeichnet, daß das Oxidationsmittel Perphthalsäure ist.

12. Verfahren gemäß einem der Ansprüche bis 11, dadurch gekennzeichnet, daß man in einem Alkanol arbeitet.

13. Verfahren gemäß einem der Ansprüche 1 bis 12, dadurch gekennzeichnet, daß die Solvolyse der Verbindung der Formel (III) eine Alkoholyse ist, die in Anwesenheit eines Alkalihydroxids oder eines Alkylicarbonats durchgeführt wird.

14. Verfahren gemäß einem der Ansprüche 1 bis 12, dadurch gekennzeichnet, daß die Solvolyse der Verbindung der Formel (III) eine Alkoholyse ist, die in Gegenwart einer wäßrigen, starken Säure durchgeführt wird.

15. Verfahren gemäß einem der Ansprüche 1 bis 14, dadurch gekennzeichnet, daß man ohne intermediäre Isolierung der Verbindung der Formel (III) arbeitet.

16. Als industrielle Verbindungen die Verbindungen der Formel (III') 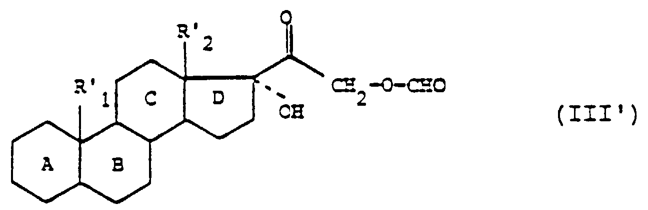 worin R'₁ ein Wasserstoffatom oder eine Methylgruppe bedeutet, R'₂ einen Methyl- oder Ethylrest bedeutet, die Ringe A, B, C und D gegebenenfalls eine oder mehrere Doppelbindungen in 1(2)-, 3(4)-, 4(5)- oder 9(11)- oder 3(4)- und 5(6)- oder 4(5)- und 6(7)- oder 1(2)- und 4(5)- oder 1,3,5(10)- oder 1(2)-, 4(5)- und 6(7)-Stellung aufweisen und gegebenenfalls durch eine oder mehrere Hydroxylfunktionen in 3-, 9- und/ oder 11-Stellung, durch eine oder zwei Ketonfunktionen in 3- und/oder 11-Stellung, durch ein oder zwei Fluor-, Chlor- oder Bromatome in 6- und/oder 9α-Stellung, durch einen oder mehrere Methyl- oder Ethylreste in 2-, 6-, 7- und/oder 16α- oder 16β-Stellung, durch einen oder zwei Methoxy- oder Ethoxyreste in 3- und/oder 11β-Stellung, durch einen Vinyl- oder Allylrest in 11β-Stellung oder durch einen Ethinylrest in 11β-Stellung substituiert sind, mit Ausnahme von Betamethason-21-formiat und der Verbindungen, worin R'₂ einen Methylrest bedeutet und entweder R'₁ für einen Methylrest steht, zwei Doppelbindungen in 1- und 4-Stellung, ein Oxorest in 3-Stellung, ein Hydroxy- oder Oxorest in 11-Stellung oder ein Hydroxyrest in 11-Stellung und ein Methylrest in 16β-Stellung vorliegen oder R'₁ für einen Methylrest steht, eine Doppelbindung in 4-Stellung, eine Ketofunktion in 3-Stellung und ein Wasserstoffatom, eine Hydroxygruppe oder eine Oxogruppe in 11-Stellung vorliegt, oder R'₁ einen Methylrest bedeutet, eine Hydroxyfunktion in 3-Stellung und eine Oxofunktion in 11-Stellung vorliegt, oder R'₁ ein Wasserstoffatom bedeutet, eine Doppelbindung in 4-Stellung und eine Ketofunktion in 3-Stellung vorliegt.

17. Als industrielle Verbindung gemäß Anspruch 16 die Verbindung der Formel

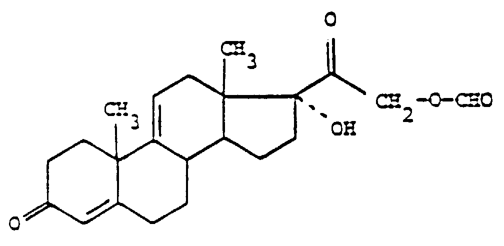

18. Als industrielle Verbindung gemäß Anspruch 16 die Verbindung der Formel

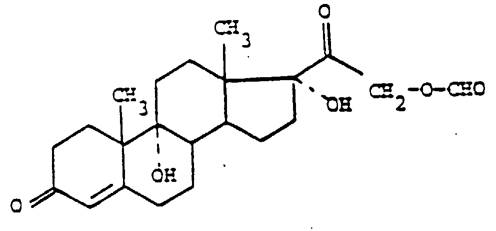

## Claims

1. Preparation process for the compounds of formula (I): 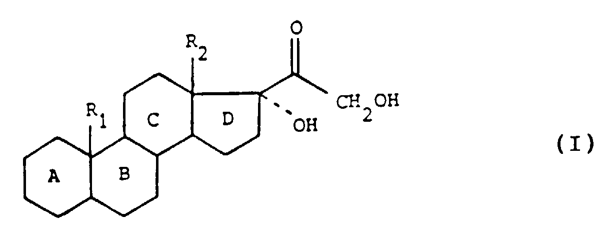 in which R₁ represents a hydrogen atom, an alkyl radical containing 1 to 4 carbon atoms, optionally substituted by a nitrogenous or oxygenated function or by a halogen atom, or R₁ represents an alkenyl or alkynyl radical containing 2 to 4 carbon atoms, R₂ represents an alkyl radical containing 1 to 4 carbon atoms, the nuclei A, B, C and D optionally carry one or more double bonds and are optionally substituted by one or more optionally protected hydroxyl or ketone functions, by one or more halogen atoms, by one or more alkyl or alkyloxy radicals containing 1 to 4 carbon atoms, or by one or more alkenyl or alkynyl radicals containing 2 to 4 carbon atoms, characterized in that a compound of formula (II): 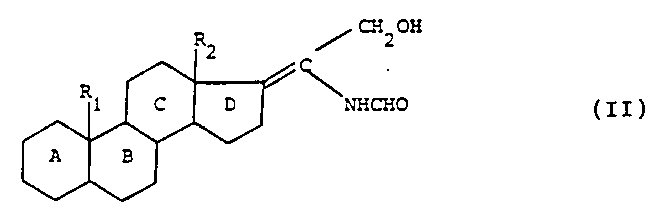 in which R₁, R₂, A, B, C and D are defined as previously, is treated with an oxidizing agent, in the presence of water, and in a solvent at least partially miscible with water, in order to obtain the compound of formula (III): 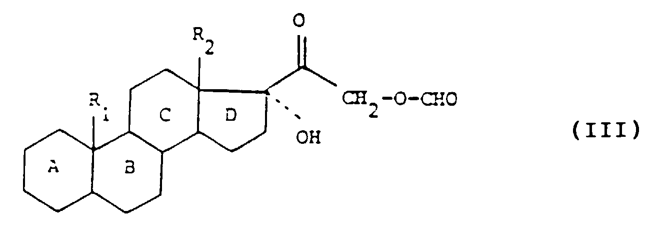 in which R₁, R₂, A, B, C and D are defined as previously, which is subjected to a solvolysis in an acidic or basic medium then, if appropriate, to a deprotection reaction of the optionally present protected hydroxyl and ketone functions.

2. Process according to claim 1, characterized in that at the start a compound of formula (II) is used corresponding to the formula (II'): 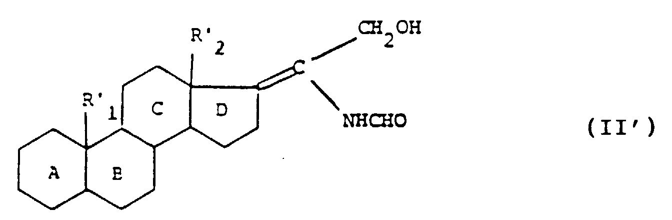 in which R'₁ represents a hydrogen atom or a methyl radical, R'₂ represents a methyl or ethyl radical, the nuclei A, B, C and D optionally carry one or more double bonds in position 1(2), 3(4), 4(5) or 9(11) or 3(4) and 5(6) or 4(5) and 6(7) or 1(2) and 4(5) or 1,3,5(10) or 1(2), 4(5) and 6(7) and are optionally substituted by one or more hydroxyl functions in position 3, 9 and/or 11, by one or two ketone functions in position 3 and/or 11, by one or two fluorine, chlorine or bromine atoms in position 6 and/or 9alpha, by one or more methyl or ethyl radicals in position 2, 6, 7 and/or 16alpha or 16beta, by one or two methoxy or ethoxy radicals in position 3 and/or 11beta, by a vinyl or allyl radical in position 11beta or by an ethynyl radical in position 11beta.

3. Process according to claim 1, characterized in that at the start a compound of formula (II) is used corresponding to the formula (II"): 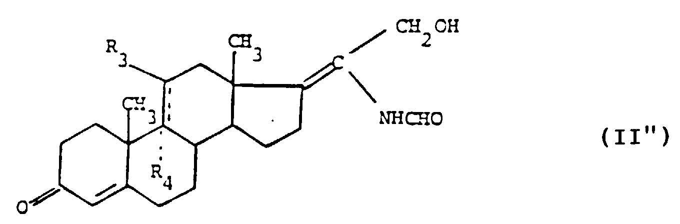 in which either the dotted line in position 9(11) represents a second bond and R₃ represents a hydrogen atom, or the dotted line does not represent a second bond and R₃ represents a hydrogen atom, a beta-hydroxy radical or an oxo radical and R₄ represents a hydrogen atom, or R₃ represents a hydrogen atom and R₄ represents an alpha-hydroxy radical.

4. Process according to any one of claims 1 to 3, characterized in that the compound of formula 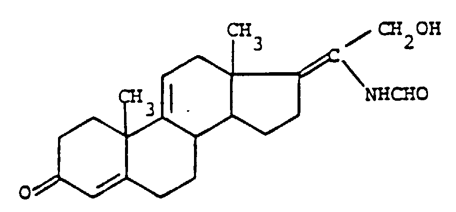 is used at the start.

5. Process according to any one of claims 1 to 3, characterized in that the compound of formula 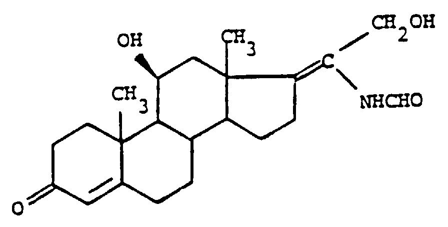 is used at the start.

6. Process according to any one of claims 1 to 3, characterized in that the compound of formula 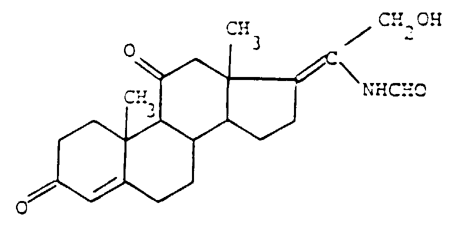 is used at the start.

7. Process according to any one of claims 1 to 3, characterized in that the compound of formula 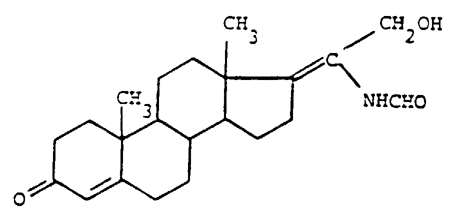 is used at the start.

8. Process according to any one of claims 1 to 3, characterized in that the compound of formula 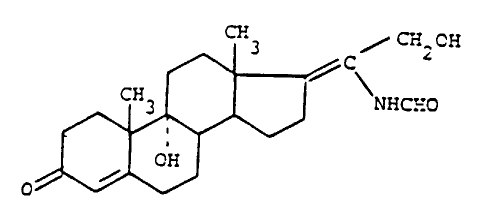 is used at the start.

9. Process according to any one of claims 1 to 8, characterized in that the oxidizing agent is chosen from the group constituted by peracids, hydrogen peroxide and hydroperoxides.

10. Process according to claim 9, characterized in that the oxidizing agent is a peracid.

11. Process according to claim 9 or 10, characterized in that the oxidizing agent is perphthalic acid.

12. Process according to any one of claims 1 to 11, characterized in that the operation is carried out in an alkanol.

13. Process according to any one of claims 1 to 12, characterized in that the solvolysis of the compound of formula (III) is an alcoholysis carried out in the presence of an alkaline hydroxide or carbonate.

14. Process according to any one of claims 1 to 12, characterized in that the solvolysis of the compound of formula (III) is an alcoholysis carried out in the presence of a strong aqueous acid.

15. Process according to any one of claims 1 to 14, characterized in that the operation is carried out without intermediate isolation of the compound of formula (III).

16. As industrial compounds, the compounds of formula (III'): 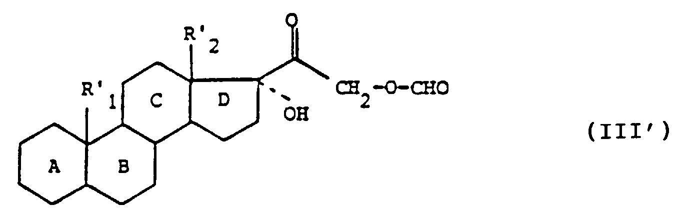 in which R'₁ represents a hydrogen atom or a methyl radical, R'₂ represents a methyl or ethyl radical, the nuclei A, B, C and D optionally carry one or more double bonds in position 1(2), 3(4), 4(5) or 9(11) or 3(4) and 5(6) or 4(5) and 6(7) or 1(2) and 4(5) or 1,3,5(10) or 1(2), 4(5) and 6(7) and are optionally substituted by one or more hydroxyl functions in position 3, 9 and/or 11, by one or two ketone functions in position 3 and/or 11, by one or two fluorine, chlorine or bromine atoms in position 6 and/or 9alpha, by one or more methyl or ethyl radicals in position 2, 6, 7 and/or 16alpha or 16beta, by one or two methoxy or ethoxy radicals in position 3 and/or 11beta, by a vinyl or allyl radical in position 11beta or by an ethynyl radical in position 11beta, with the exception of the betamethasone 21-formate and of the compounds in which R'₂ represents a methyl radical and either R'₁ represents a methyl radical, two double bonds are present in position 1 and 4, an oxo radical in position 3, a hydroxy or oxo radical in position 11 or a hydroxy radical in position 11 and a methyl radical in position 16beta, or R'₁ represents a methyl radical, a double bond is present in position 4, a keto function in position 3 and a hydrogen atom, a hydroxy radical or an oxo radical in position 11, or R'₁ represents a methyl radical, a hydroxy function is present in position 3 and an oxo function in position 11, or R'₁ represents a hydrogen atom, a double bond is present in position 4 and a keto function in position 3.

17. As an industrial compound, according to claim 16, the compound of formula:

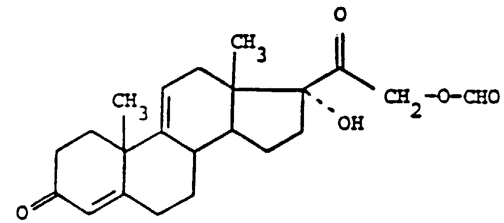

18. As an industrial compound, according to claim 16, the compound of formula:
